# EUROPEAN PATENT APPLICATION

(11) **EP 4 134 126 A1**
(43) Date of publication of application: **15.02.2023**
(21) Application number: 22188638.5
(22) Date of filing: 04.08.2022
(51) Int. Cl.: A61N 1/36

(54) **NEUROMODULATION SYSTEM AND METHOD**

(30) Priority: 11.08.2021 US 202117399780
(71) Applicant: ONWARD Medical N.V., 5656 AE Eindhoven (NL)
(72) Inventor: Delattre, Vincent, 5656 AE Eindhoven (NL); Subbaroyan, Jeyakumar, 5656 AE Eindhoven (NL); Harari, David, 5656 AE Eindhoven (NL); Moritz, Chet, 5656 AE Eindhoven (NL)
(74) Representative: DTS Patent- und Rechtsanwälte Schnekenbühl und Partner mbB

(57) **Abstract**

The disclosed systems and methods include a neuromodulation system including at least one neuromodulation device, at least one neuromodulation pattern storage means, and at least one neuromodulation controller. The neuromodulation pattern storage means can store neuromodulation data. The neuromodulation data can specify neurostimulation with at least one of a carrying frequency of at least 1 kHz or multipolar stimulation. The neuromodulation device can provide neuromodulation according to the neuromodulation data.

## Description

### TECHNICAL FIELD

Some applications of the present invention relate to systems and methods for providing electrical neuromodulation. More specifically, some applications of the present invention relate to the use of systems for providing neuromodulation in methods for at least one of the treatment, prevention, or relief of pain of a subject equipped with the system, as well as methods for the treatment, prevention, and/or relief of pain.

### BACKGROUND

A spinal cord injury may interrupt the communication between the spinal cord and supraspinal motion control centers, depriving spinal sensorimotor circuits of the excitatory and modulatory drives necessary to produce movement.

Electrical neuromodulation of the lumbar spinal cord using epidural electrical stimulation (EES) can (re-)activating such spinal sensorimotor circuits. For example, EES has restored coordinated locomotion in animal models of spinal cord injuries, and isolated leg movements in individuals with motor paralysis. A neuromodulation system can be used to provide such EES. For example, a system can deliver adaptive electrical spinal cord stimulation to facilitate and restore locomotion after neuromotor impairment. Likewise, a system can provide selective spatiotemporal EES of the spinal cord. However, unpleasant side effects (e.g., pain, discomfort, paresthesia, or the like) can limit the clinically applicability of EES.

### SUMMARY

Disclosed systems and methods provide neuromodulation configured to reduce or potentially eliminate unpleasant EES side effects. The embodiments of the present disclosure include systems and methods for providing neuromodulation for the treatment, prevention, and/or the relief of pain of a subject.

Consistent with some embodiments of the present disclosure, a neuromodulation system is provided. The system may include at least one neuromodulation device, at least one neuromodulation pattern storage means, and at least one neuromodulation controller. In some embodiments, the neuromodulation pattern storage means may include neuromodulation data, and the neuromodulation device may provide neuromodulation according to the neuromodulation data. The neuromodulation data may include data related to a carrying frequency of at least 1 kHz and/or data related to multipolar stimulation.

Additional objects and advantages of the embodiments will be set forth in part in the description that follows, and in part will be obvious from the description or may be learned by practice of the embodiments. The objects and advantages of the embodiments will be realized and attained by means of the elements and combinations particularly pointed out in the appended claims.

It is to be understood that both the foregoing general description and the following detailed description are exemplary and explanatory only and are not restrictive of the claims.

### BRIEF DESCRIPTION OF THE DRAWINGS

The drawings are not necessarily to scale or exhaustive. Instead, emphasis is generally placed upon illustrating the principles of the inventions described herein. The accompanying drawings, which are incorporated in and constitute a part of this specification, illustrate several embodiments consistent with the disclosure and together with the description, serve to explain the principles of the disclosure. In the drawings:
**FIG. 1** illustrates an exemplary system for providing neuromodulation, consistent with some embodiments of the present disclosure.
**FIG. 2** illustrates a flow chart of an exemplary method the treatment, prevention, and/or relief of pain of a subject, consistent with some embodiments of the present disclosure.
**FIGS. 3A** and **3B** depict exemplary placement of EES electrodes, consistent with some embodiments of the present disclosure.
**FIG. 4** depicts an exemplary method for configuration of EES stimulation to treat a neurological disease, dysfunction, or injury in a patient, consistent with disclosed embodiments.
**FIG. 5** depicts exemplary stimulation patterns for use in EES, consistent with some embodiments of the present disclosure.
**FIG. 6A** depicts a trace of an additional exemplary stimulation pattern for use in EES, consistent with some embodiments of the present disclosure.
**FIG. 6B** depicts an illustration of a further exemplary stimulation pattern for use in EES, consistent with some embodiments of the present disclosure.

### DETAILED DESCRIPTION

Exemplary embodiments are described with reference to the accompanying drawings. While examples and features of disclosed principles are described herein, modifications, adaptations, and other implementations are possible without departing from the spirit and scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It should also be noted that as used herein and in the appended claims, the singular forms "a," "an," and "the" include plural references unless the context clearly dictates otherwise.

In the following description, various working examples are provided for illustrative purposes. However, is to be understood the present disclosure may be practiced without one or more of these details.

This disclosure is provided for the convenience of the reader to provide a basic understanding of a few exemplary embodiments and does not wholly define the breadth of the disclosure. This disclosure is not an extensive overview of all contemplated embodiments and is intended to neither identify key or critical elements of all embodiments nor to delineate the scope of any or all aspects. Its sole purpose is to present some features of one or more embodiments in a simplified form as a prelude to the more detailed description presented later. For convenience, the term "disclosed embodiments" or "exemplary embodiment" may be used herein to refer to a single embodiment or multiple embodiments of the disclosure.

In accordance with disclosed embodiments, EES can be applied to a subject using a carrying frequency (e.g., a carrying frequency of at least 1 kHz), and/or multipolar stimulation. Such application may reduce or potentially eliminate side unpleasant EES side effects. For example, a stimulation partiture using a carrying frequency and/or multipolar stimulation to evoke a desired motor response in a subject may exhibit a reduction or elimination of unpleasant side effects, as compared to conventional stimulation.

In some embodiments, a neuromodulation system may provide frequency neuromodulation on top of the carrying frequency. In some embodiments, the carrying frequency may be between 1 and 15 kHz, between 1 and 10 kHz, and/or between 1 and 4.5 kHz.

In some embodiments, an exemplary neuromodulation system may be used for treating a subject suffering from a neurological disease, dysfunction, or injury. Such a neurological disease, disorder, or injury can include at least one of stroke, trauma, spinal cord injury, tumor, Parkinson's disease, Alzheimer's disease, neurodegenerative disease, or chronic pain conditions. In some embodiments, a chronic pain condition can include at least one of low back pain from failed back surgery syndrome (FBSS) or non-operable conditions, refractory angina, painful diabetic neuropathy, complex regional pain syndrome, post-surgical pain, post-amputation pain, abdominal pain, pelvic pain, or critical limb ischemia. In some embodiments, a neurological disease, dysfunction, or injury can include at least one of motor dysfunction, autonomic dysfunction, bladder dysfunction, bowel dysfunction, or sexual dysfunction (e.g., secondary to at least one of stroke, trauma, spinal cord injury, tumor, Parkinson's disease, Alzheimer's disease or neurodegenerative disease). In other words, in some applications, a subject may be treated with the system to restore at least one of motor function, autonomic function, bladder function, bowel function, or sexual function.

An exemplary neuromodulation system may include at least one neuromodulation controller. A controller may be any physical device or group of devices having electric circuitry that performs a logic operation on input or inputs. For example, the at least one neuromodulation controller may include one or more integrated circuits (IC), including application-specific integrated circuit (ASIC), microchips, microcontrollers, microprocessors, all or part of a central processing unit (CPU), graphics processing unit (GPU), digital signal processor (DSP), field-programmable gate array (FPGA), server, virtual server, or other circuits suitable for executing instructions or performing logic operations. The instructions executed by at least one neuromodulation controller may, for example, be pre-loaded into a memory integrated with or embedded into the controller or may be stored in a separate memory. The at least one neuromodulation controller may be configured to control and/or program the neuromodulation device.

An exemplary neuromodulation system may include at least one neuromodulation pattern storage means. Such a storage means may include at least one memory, such as a Random-Access Memory (RAM), a Read-Only Memory (ROM), a hard disk, an optical disk, a magnetic medium, a flash memory, other permanent, fixed, volatile or non-volatile memory, or any other mechanism capable of storing information and/or instructions. The memory may include one or more separate storage devices collocated or disbursed, capable of storing data structures, instructions, or any other data. In some embodiments, the storage means may be integrated or embedded in the neuromodulation controller. The storage means can be configured to provide the neuromodulation device with neuromodulation data. For example, the neuromodulation applied by the neuromodulation device may be defined and/or provided by the neuromodulation data. Additionally or alternatively, the neuromodulation data may include data related to stimulation parameters (e.g., stimulation frequency, stimulation amplitude, stimulation current, stimulation pulse width, electrode configuration, or the like).

In some embodiments, the neuromodulation system may provide at least one of tonic, burst, or high-frequency EES. In some embodiments, the neuromodulation system may provide EES having shaped pulses (e.g., ramp, exponential, sawtooth, or the like pulse shapes). In some embodiments, tonic EES may be provided at stimulation frequencies between 1 and 125 Hz, although other frequencies may be used. In some embodiments, burst EES may include pulse sequences (e.g., pairs, triplets, quadruplets, quintuplets, or the like). The pulses within the pulse sequence can be provided at an interpulse frequency (e.g., at least 200 Hz, between 300 and 750 Hz, or other suitable frequencies) and the pulse sequences can be provided at an interburst frequency (e.g., between 1 and 125 Hz, between 10 and 60 Hz, or other suitable interburst frequencies). The disclosed embodiments are not limited to these disclosed frequencies and/or burst patterns. High frequency EES may be or include pulse trains or continuous stimulation waveforms. In some embodiments, a frequency of such a pulse train or continuous stimulation waveform can be between 200 Hz to 10 kHz, although other frequencies may be used.

An exemplary neuromodulation device may include a neurostimulation device, in particular a pulse generator, for example an implantable pulse generator. Additionally or alternatively, the pulse generator may be a non-implantable pulse generator.

In some embodiments, an exemplary neuromodulation device may include at least one of a sensor, a programmer, a powering device, a lead including at least one electrode (e.g., an implantable device), a communication module, and/or a telemetry module.

A sensor may include at least one of an inertial measurement unit, an optical sensor, a camera, a piezo element, a velocity sensor, an accelerometer, a magnetic sensor, a torque sensor, a pressure sensor, a displacement sensor, a contact sensor, an EMG measurement unit, an electroneurogram measurement unit, a goniometer, a Hall sensor and/or a gyroscope and/or motion tracking video camera, or infra-red camera, or any other electronic component configured to provide feedback information about the neuromodulation provided.

An exemplary neuromodulation system may include at least one open-loop system, closed-loop system, pseudo-open-loop system, or any combination thereof.

A programmer may include an application installed on at least one device that communicates with the at least one neuromodulation controller. The at least one device may include at least one interface component, such as a graphical user interface, that allows a user to perform operations such as selecting, starting, and/or stopping a task or otherwise configuring stimulation parameters through the programmer. Consistent with disclosed embodiments, the programmer may be used by a user (e.g., a physician, therapist, physiotherapist, patient, or other suitable user) to provide inputs to the neuromodulation controller (e.g., inputs selecting, starting, or stopping a task; inputs configuring stimulation parameters; or other suitable inputs).

In some embodiments, a programmer may be configured to allow adjusting the stimulation parameters of a task while the task is running. Advantageously, this enables the user to tune the stimulation without having to start and stop the task, which may be very cumbersome at the start of the rehabilitation training when all stimulation partitures are developed and tuned.

In some embodiments, a programmer may include, but is not limited to, a physiotherapist programmer (PTP), patient programmer (PP), or any other application installed on at least one user device that communicates with the neuromodulation controller.

A lead may include multiple electrodes. For example, in some embodiments, a lead may include 2, 16, or 32 electrodes, although any number of electrodes greater than one may be used. For epidural electrical stimulation, the lead may be positioned in the epidural space (i.e., on the outside of the dural sac, which encases the spinal cord and the cerebrospinal fluid in which the spinal cord 'floats'), on top of the spinal cord (e.g., proximate to spinal segments T12, L1, L2, L3, L4, L5, S1, or another spinal segment or segments, based on the desired therapeutic effect).

In some embodiments, multipolar stimulation may use a multipolar electrode configuration. In such a configuration, EES can be provided using multiple (i.e., two or more) electrodes. In some instances, multipolar stimulation can be provided using a selected subset of the electrodes within the lead (e.g., when the lead includes more electrodes than those used in the multipolar stimulation).

The powering device may include any element or circuit enabled to accumulate energy and/ provide energy to the system or any component thereof. Non-limiting examples include capacitors, supercapacitors, batteries, receiver coils (e.g., radiofrequency receiver coils), or the like. In some embodiments, the powering device can include an energy storage component for storing power. The powering device can be configured to receive power from an external source, store the power in the energy storage component, and provide the power as needed for use by other circuits, components, or devices of the neuromodulation system. For example, the powering device may be or may include at least one battery, for instance a rechargeable battery.

A communication module and a telemetry module may be configured enable wireless communication between at least two subsystems of the system. For example, a communication and/or telemetry module may include an antenna (e.g., a structure configured to transmit or receive electromagnetic waves). For example, an antenna may include one or more conducting elements and/or non-conducting dielectric elements arranged in a manner enabling the transmission and/or reception of radio signals, or any other component and/or device configured for receiving and/or transmitting energy from the air or from any other medium in which the antenna is placed. The antenna may also be electrically coupled to at least one separate receiver and/or transmitter, directly or wirelessly. In some embodiments, the antenna can be part of the powering device (e.g., the antenna could be used for communication as well as receiving power).

In some embodiments, an exemplary neuromodulation system may include, for example, a system for transcutaneous electrical stimulation, epidural electrical stimulation, subdural electrical stimulation, functional electrical stimulation, intramuscular stimulation, and/or dorsal root ganglion stimulation. In particular, in some embodiments, it may be generally possible that the system is a central nervous system (CNS) stimulation system and/or peripheral nervous system stimulation (PNS) system.

Some embodiments of the present disclosure relate to a method for the treatment and/or prevention and/or relief of pain of a subject. The method may include, for example, equipping a patient with a neuromodulation device, providing neuromodulation data, and providing neuromodulation to the patient according to the neuromodulation data. In some embodiments, the neuromodulation data may include data related to a carrying frequency of at least 1 kHz and/or data related to multipolar stimulation.

Some embodiments of the present disclosure relate to the use of a neuromodulation system in a method for the treatment and/or prevention and/or the relief of neuromodulation-related pain or discomfort of a subject equipped with the system.

Further details and advantages of some exemplary embodiments shall now be discussed with reference to the drawings.

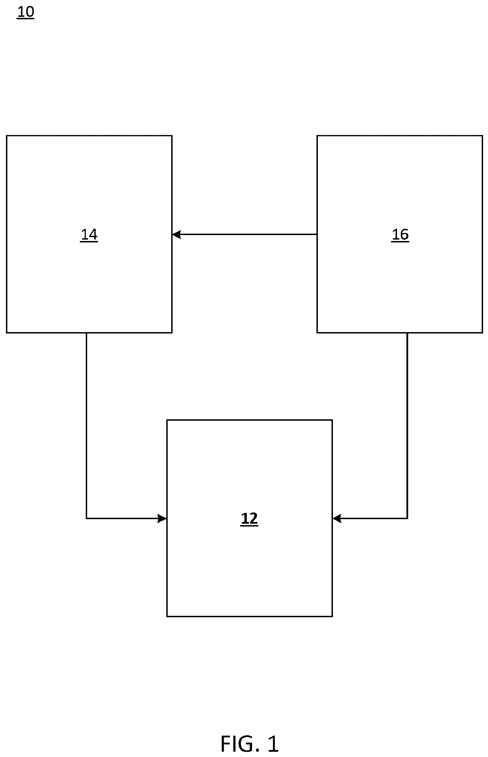

By way of example, **FIG. 1** illustrates an exemplary embodiment of the system 10 for providing neuromodulation, consistent with the present disclosure. In this exemplary embodiment, the system 10 may include neuromodulation device 12. In some embodiments, neuromodulation device 12 may include a neurostimulation device, such as an implantable pulse generator. Although only one neuromodulation device 12 is illustrated herein, it is to be understood that system 10 may include any number of neuromodulation devices, consistent with the present disclosure.

In some embodiments, system 10 may include neuromodulation pattern storage means 14. Neuromodulation pattern storage means 14 at least one memory, such as a Random-Access Memory (RAM), a Read-Only Memory (ROM), a hard disk, an optical disk, a magnetic medium, a flash memory, other permanent, fixed, volatile or non-volatile memory, or any other mechanism capable of storing information and/or instructions. Although only one neuromodulation pattern storage means 14 is illustrated herein, it is to be understood that system 10 may include any number of neuromodulation pattern storage means, consistent with the present disclosure.

In some embodiments, system 10 may include a neuromodulation controller 16. Neuromodulation controller 16 may include any physical device or group of devices having electric circuitry that performs a logic operation on input or inputs. For example, the at least one neuromodulation controller may include one or more integrated circuits (IC), including application-specific integrated circuit (ASIC), microchips, microcontrollers, microprocessors, all or part of a central processing unit (CPU), graphics processing unit (GPU), digital signal processor (DSP), field-programmable gate array (FPGA), server, virtual server, or other circuits suitable for executing instructions or performing logic operations. Although only one neuromodulation controller 16 is illustrated herein, it is to be understood that system 10 may include any number of neuromodulation controllers, consistent with the present disclosure.

In some embodiments, neuromodulation pattern storage means 14 may be integrated or imbedded in neuromodulation controller 16, or neuromodulation controller 16 may be integrated or embedded in neuromodulation pattern storage means 14.

Neuromodulation pattern storage means 14 may be connected to neuromodulation device 12. In some embodiments, the connection between neuromodulation pattern storage means 14 and neuromodulation device 12 may be a direct connection or an indirect connection.

In some embodiments, the connection between neuromodulation pattern storage means 14 and the neuromodulation device 12 may be a unidirectional connection. For example, the connection may be unidirectional from neuromodulation pattern storage means 14 to neuromodulation device 12, or it may be unidirectional from neuromodulation device 12 to neuromodulation pattern storage means 14.

In some embodiments, a bidirectional connection between one or more components of system 10 may be possible. For example, in some embodiments, the connection between neuromodulation pattern storage means 14 and neuromodulation device 12 may be a wireless connection or a cable-bound connection.

Neuromodulation controller 16 may be connected to neuromodulation pattern storage means 14. In some embodiments, the connection between the neuromodulation controller 16 and the neuromodulation pattern storage means 14 may be a direct connection or an indirect connection.

In some embodiments, the connection between neuromodulation controller 16 and the neuromodulation pattern storage means 14 may be a unidirectional connection. For example, the connection may be unidirectional from neuromodulation controller 16 to neuromodulation pattern storage means 14, or it may be unidirectional from neuromodulation pattern storage means 14 to the neuromodulation controller 16.

In some embodiments, a bidirectional connection between one or more components of system 10 may be possible. For example, in some embodiments, the connection between the neuromodulation controller 16 and the neuromodulation pattern storage means 14 may be a wireless connection or a cable-bound connection.

Neuromodulation controller 16 may be connected to the neuromodulation device 12.

In some embodiments, the connection between neuromodulation controller 16 and neuromodulation device 12 may be a direct connection or an indirect connection.

In some embodiments, the connection between the neuromodulation controller 16 and the neuromodulation device 12 may be a unidirectional connection. For example, the connection may be unidirectional from neuromodulation controller 16 to the neuromodulation device 12, or it may be unidirectional from neuromodulation device 12 to neuromodulation controller 16.

In some embodiments, a bidirectional connection between one or more components of system 10 may be possible. For example, the connection between neuromodulation controller 16 and neuromodulation device 12 is a wireless connection or a cable-bound connection.

Neuromodulation pattern storage means 14 may be configured to store neuromodulation data. In some embodiments, the neuromodulation data may be transferred from neuromodulation pattern storage means 14 to neuromodulation device 12. In some embodiments, the neuromodulation data may include data related to a carrying frequency of at least 1 kHz and/or data related to multipolar stimulation. For example, the carrying frequency may be between 1 and 15 kHz, between 1 and 10 kHz, and/or preferably between 1 and 4.5 kHz. In some embodiments, neuromodulation device 12 may provide neuromodulation according to the neuromodulation data.

In some embodiments neuromodulation controller 16 may control and/or program neuromodulation device 12, for example via the neuromodulation pattern storage means 14.

In some embodiments, system 10 may include at least one of a sensor, a programmer, a powering device, a lead comprising at least one electrode, a communication module, a telemetry module. In some embodiments, system 10 may be a system for transcutaneous electrical stimulation, epidural electrical stimulation, subdural electrical stimulation, functional electrical stimulation, intramuscular stimulation, and/or dorsal root ganglion stimulation.

In some embodiments, the stimulation data stored in neuromodulation pattern storage means 14 may include data related to at least one of the parameters stimulation frequency, stimulation amplitude, stimulation current, pulse width, and/or electrode configuration.

In some embodiments, system 10 may be used in a method for the treatment, prevention, and/or the relief of pain of a subject equipped with the system 10. The pain to be relieved, for example, may be pain that is typically caused and/or induced by neuromodulation.

The subject of disclosed neuromodulation methods may, for example, be a patient suffering from chronic pain, motor dysfunction, autonomic dysfunction, bladder dysfunction, bowel dysfunction and/or sexual dysfunction.

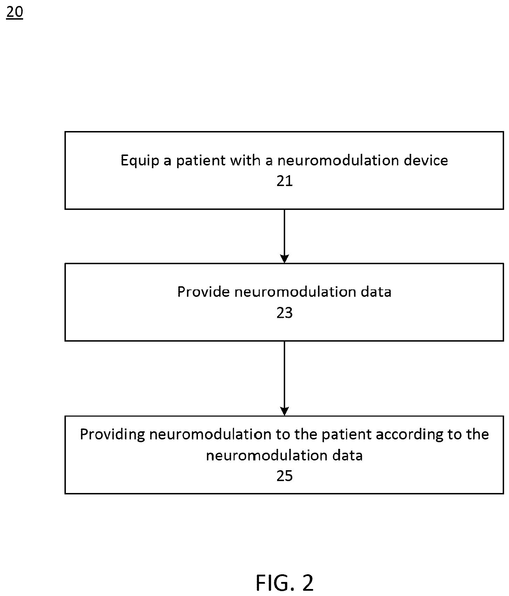

In some embodiments system 10 may be at least partially configured and arranged for performing neuromodulation methods, as illustrated for example in **FIG. 2****.**

By way of example, **FIG. 2** illustrates an exemplary embodiment of a method 20 for the treatment, prevention, and/or relief of pain of a subject, consistent with the present disclosure.

In some embodiments, method 20 may include steps 21 to 25, although additional and/or intermediate steps may be possible. In step 21 of exemplary method 20, a patient is equipped with neuromodulation device 12. In step 23, neuromodulation data are provided to neuromodulation device 12. In step 25, neuromodulation is provided to the patient according to the neuromodulation data, for example via the neuromodulation device 12.

In some embodiments, the neuromodulation data provided in step 23 can be provided in response to a determination that the subject experiences unpleasant neuromodulation-related side effects, as described herein. For example, the subject may be using conventional stimulation and may be experiencing pain, discomfort, and/or paresthesia effects as a result of the conventional stimulation. The neuromodulation data can be provided in response to this determination.

In some embodiments, the neuromodulation data provided in step 23 can be provided in response to a determination that the subject experiences at least one of motor dysfunction, autonomic dysfunction, respiratory dysfunction, blood pressure dysfunction, bladder dysfunction, bowel dysfunction, sexual dysfunction, or chronic pain. The neuromodulation data may be provided in response to this determination.

In some embodiments, the neuromodulation data provided to neuromodulation device 12 at step 23 may include data related to a carrying frequency of at least 1 kHz and/or data related to multipolar stimulation. For example, the carrying frequency may be between 1 and 15 kHz, between 1 and 10 kHz, and/or between 1 and 4.5 kHz. In some embodiments, the neuromodulation data may be related to at least one of the parameters stimulation frequency, stimulation amplitude, stimulation current, pulse width, and/or electrode configuration.

In some embodiments, a non-transitory computer-readable storage medium including instructions is also provided, and the instructions may be executed by a device (such as the disclosed encoder and decoder), for performing the above-described methods. Common forms of non-transitory media include, for example, a floppy disk, a flexible disk, hard disk, solid state drive, magnetic tape, or any other magnetic data storage medium, a CD-ROM, any other optical data storage medium, any physical medium with patterns of holes, a RAM, a PROM, and EPROM, a FLASH-EPROM or any other flash memory, NVRAM, a cache, a register, any other memory chip or cartridge, and networked versions of the same. The device may include one or more processors (CPUs), an input/output interface, a network interface, and/or a memory.

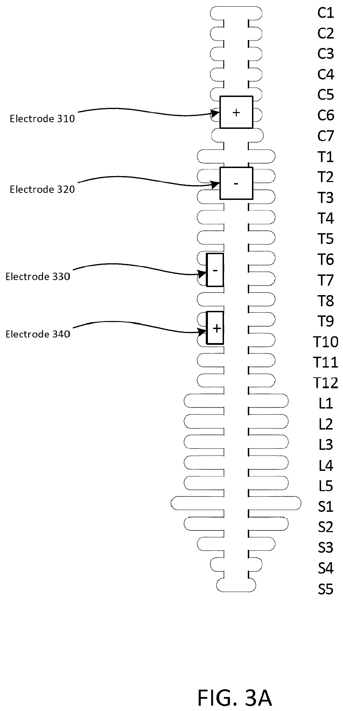

FIG. 3A depicts a simplified coronal schematic of the spine, showing the vertebral levels C1 to S5, with the spinal roots indicated as projections, consistent with disclosed embodiments. Exemplary electrode placements suitable for EES are depicted, consistent with disclosed embodiments. In a first exemplary placement, electrode 310 can be placed above a location to be stimulated and electrode 320 can be placed below the location to be stimulated. As depicted, electode 310 and electrode 320 can be configured to provide biopolar stimulation. Such a configuration may localize the effect of stimulation to the region between the electrodes. In some embodiments, one or more of electtode 310 or electrode 320 can be configured to provide monopolar stimulation (e.g., stimulation current can be returned through a remote electrode, the can of the stimulator, or according to other known methods). In some instances, monopolar stimulation may affect or activate target structures, such as neurons, in a broader area. The first exemplary placement depicts a midline placement of electrode 310 and electrode 320. In a second exemplary placement, electrode 330 can be placed above a location to be stimulated and offset to one side of the spinal canal. Electode 340 can be placed below the location to be stimulated and offset to the same side of the spinal canal (in some embodiments, not shown, electrode 340 can be placed on the contralateral side of the spinal canal). In such lateral embodiments, activation thresholds for certain target structures (e.g., the dorsal roots or dorsal root ganglions, or the like) can be reduced. However, the amplitude threshold for evoking unpleasent or painful sensations may be reduced as well.

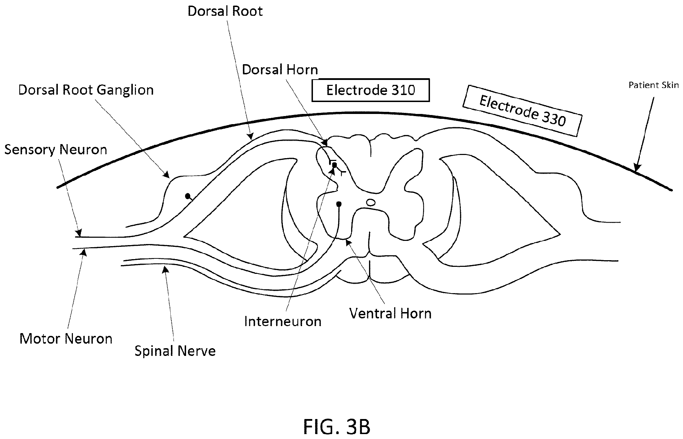

**FIG. 3B** depicts a transverse schematic of the spinal cord, consistent with disclosed embodiments. As depicted in **FIG. 3B****,** the electrodes can be positioned, in some embodiments, above the dorsal spinal cord. In some embodiments, the electrodes can be positioned on the skin of the patient and configured to provide transcutaneous stimulation. In some embodiments, the electrodes can be positioned extradurally (the spinal canal and dura are not depicted in **FIG. 3B****).** In some embodiments, the electrodes can be positioned subdurally. The course and location of the cell bodies (e.g., in the dorsal root ganglion and ventrial horn) of the sensory and motor neurons are depicted in **FIG. 3B****.** As shown, the position of electrode 310 may support bilateral activation of the dorsal roots (or stimulation of the dorsal columns or dorsal horn). Likewise the position of electrode 330 (depicted in this example as projected into transverse plane including electrode 310) can support unilateral activation of the dorsal roots. As may be appreciated, the disclosed positions are exemplary: a typical lead may include more electrodes, differently sized electrode, differently placed electrode, or differ in other ways from the configuration depicted in **FIGs. 3A** and **3B****.**

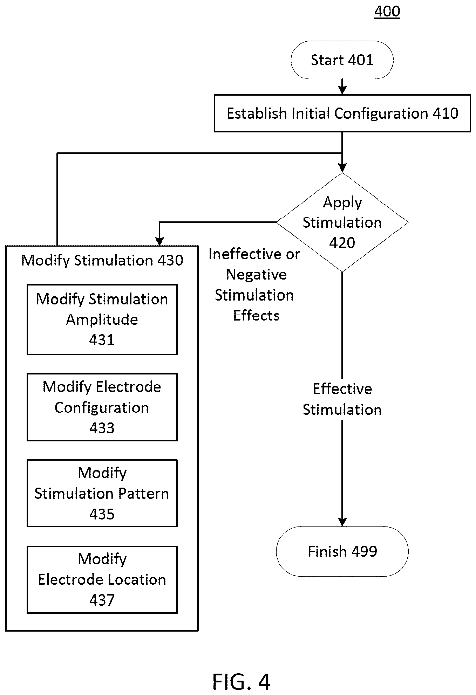

**FIG. 4** depicts an exemplary method 400 for configuration of EES stimulation to treat a neurological disease, dysfunction, or injury in a patient, consistent with disclosed embodiments. In some embodiments, method 400 can be used with a patient implanted (e.g., intraoperatively or post-operatively, or the like) with electrodes for EES stimulation (e.g., using neuromodulation device 12 of system 10, or the like). In various embodiments, method 400 can be used with external, non-implanted electrodes (for example for transcutaneous stimulation).

In step 410 of method 400, after beginning in step 401, an initial EES configuration can be established. The initial configuration can include an electrode location (or activation location), a stimulation pattern, an electode configuration, or a stimulation amplitude.

Establishing the initial configuration can include implanting the electrodes. The electrodes can be implemented at a predetermined position. The predetermined position can depend on the neuroanatomy of the particular patient being implanted, on the typical neuroanatomy of a demographic group to which the patient belongs, or on general neuroanotomical principles. For example, based on general neuroanotomical principles, the spinal roots exiting the spinal canal between segments L1 to L5 may be assumed to innervate, among other structures, the colon. Treatment for constipation, colitis, diarrhea, gas pain, irritable bowel, or similar disorders may therefore involve placing on the skin (e.g., for transcutaneuous stimulation) or implanting electrodes above and below one or more of these segments (e.g., at L1 and L3, targeting the spinal roots exiting at L2; or at L2 and L5, targeting the spinal roots exiting a L3 and L4, or the like). Alternatively, a prior intervention with a particular patient may have established that stimulation to treat constipation should target the spinal roots exiting at S1 (or another segment). As an additional example, when the treatment is intended to address orthostatic hypotension, the expected location can be around T10 to T12. Accordingly, electrodes can be placed on the skin (e.g., for transcutaneuous stimulation) or implanted at T10 and T12. As a further example, for bladder function, the expected location can be anywhere between T12 through L5.

Consistent with disclosed embodiments, leads can include electrodes 310 to 340. The leads can be single electrode leads or multi-electrode leads, as described herein. For example, electrodes 310 and 320 can be components of a multi-electrode lead. As an additional example, electrodes 330 and 340 can each be part of a corresponding single electrode lead. In some embodiments, the electrodes can be round, square, or rectangular. In various embodiments the electrodes can have a diameter (or side length) up to 2 inches. For example, the electrodes can be adhesive carbonized sillicone rubber electrodes, metal electrodes (e.g., silver, stainless steel, tantalum, or platinum contact or metal fabric electrodes), or the like. The disclosed embodiments are not limited to any particular type of lead. In some embodiments, the electrodes can be adapted for transcutaneous stimulation. In some embodiments using two or more electrodes, the location of activation can be adjusted (e.g., using current steering or the like) without adjusting the positioning of the electrodes.

Establishing the initial configuration can include configuring a stimulator (e.g., an implantable pulse generator of neuromodulation device 12) to provide stimulus according to a stimulus pattern. In some embodments, the stimulus pattern can specify an overall duration of the stimulus, an intraburst interval, a number of pulses in each burst in the stimulus, a pulse amplitude (or relative pulse amplitude) for each pulse, a pulse shape for each pulse, or other stimulation pattern parameters.

Establishing the initial configuration can include establishing an electrode configuration. The electrode configuration can include whether the stimulation is multi-polar or monopolar (e.g., with a remote electrode or the can of neuromodulation device 12 serving as a return electrode). In some embodiments, the electrode configuration can include an allocation of current (e.g., an allocation of the total stimulation current) among multiple electrodes in a multi-electrode configuration (e.g., thus implementing current steering, or the like).

Establishing the initial configuration can include establishing an inital stimulation amplitude. The disclosed embodiments are not limited to any particular manner of specifying the stimulation amplitude. In some embodiments, the stimuluation pattern may specify relative amplitudes. These relative amplitudes can be scaled by a scaling factor to generate the amplitudes of the stimulation applied to the patient. In such embodiments, adjusting the scaling factor can specify the amplitude of the stimulation applied to the patient. In various embodiments, different versions of the stimulation pattern can be saved, each version corresponding to a different stimulation amplitude. In some embodiments, multiple amplitude parameters can describe a stimulation pattern. These amplitude parameters may be independently variable. In such embodiments, the stimulation amplitude may be a vector of the parameters defining the amplitudes in the stimulation pattern.

After establishing an initial EES configuration in step 410, method 400 can procede to step 420. In step 420, EES stimulation can be applied using the implanted electrodes. A measure of treatment effectiveness can also be acquired. For example, when the EES stimulation is provided to treat chronic pain, the patient can provide a report of pain during (or after) stimulation. When the EES stimulation is provided to combat orthostatic hypotension, a measure of blood pressure following a postural change can be acquired. Any negative stimulation effects can also be measured. Such negative stimulation effects can include pain, discomfort, parathesia, unintended movements, or other EES side effects.

When the EES stimulation evokes a desired therapeutic response, method 400 can finish successfully at step 499. The criteria for effective stimulation can depend on the EES stimulation type. For example, EES for treatment of pain may have a different criteria for effective stimulation than EES for the treatment of motor dysfunction. When the EES stimulation does not evoke the desired therapeutic response or evoke unacceptable negative stimulation effects, method 400 can transition to step 430.

In step 430, the stimulation provided to the patient can be modified. Exemplary modifications can include modifying the electrode location 437, modifying the stimulation pattern 435, modifying the electrode configuration 433, or modifying the electrode amplitude 431.

Modifying the electrode amplitude 431 can include increasing or decreasing the amplitude of the stimulation. For example, when the stimulation evokes negative stimulation effects, modifying the electrode amplitude 431 can include decreasing the amplitude of stimulation. As an additional example, when the stimulation evokes an ineffective response (or no response at all), modifying the electrode amplitude 433 can include increasing the amplitude of the stimulation.

Modifying the electrode configuration 433 can include converting from monopolar stimulation to multi-polar stimulation, changing the stimulating electrodes on a lead (or swaping the anode and cathode, or the like), changing the distribution of currents between electrodes, or other changes to the electrode configuration. The electrode configuration can be changed from monopolar stimulation to multi-polar stimulation in response to indicia of excessive or undesirable stimulation of non-target structures. Such indicia can include movement (e.g., muscle contractions or spasms during stimulation), pain, parathesia, activation of non-target neuroanatomical functionalities (e.g., increases in blood pressure, sweating, or other autonomic responses), or the like. Switching to multi-polar stimulation can increase the selectivity or targeting of stimulation. Similarly, by changing the electrodes in a multi-electrode lead that are providing stimulation (or changing the relative weightings of current provided by each electrode), the location targeted for activation can be changed. Alternatively, switching from multi-polar stimulation to monopolar stimulation can reduce the power required to activate a target structure (e.g., therefore reducing the potential for tissue damage or extending battery life).

Modifying the stimulation pattern 435 can include changing a configuration of the stimulator to provide stimulus according to a modified stimulus pattern. In some embodments, modifications to the stimulus plan can affect an overall duration of the stimulus, an intraburst interval, a number of pulses in each burst in the stimulus, a pulse amplitude (or relative pulse amplitude) for each pulse, a pulse shape for each pulse, or other stimulation pattern parameters. For example, decreasing the interpulse interval can increase the capacitive bypassing of the skin impedance and reduce the activation of skin nociceptor (e.g., thus reducing unpleasant sensations or pain in the patient). However, decreasing the interpulse interval can require corresponding decreases to the pulse duration. Decreases to the pulse duration can increase the stimulation amplitudes required to activate target structures, increasing the potential for stimulation evoked tissue damage or electrode corrosion and also increasing the power requirements of the stimulator.

Modifying the electrode location 437 can include adjusting the position of one or more electrodes to improve treatment outcomes. In some embodiments using implanted electrodes, the adjustment can occur interoperatively. In various embodiments using transcutaneous electrodes, the electrodes can be repositioned or replaced as needed. In some embodiments, a treatment can be applied using a particular position (or location of activation) and a treatment effect can be measured. Based on the measured treatment effect, the position (or location of activation) can be updated.

The disclosed embodiments are not limited to any particular ordering of different types of modifications. In some embodiments, modifications to stimulation amplitude may preceed modifications to electrode configurations. For example, EES can be provided using progressively greater stimulation amplitudes until effective stimulation is achieved or unacceptable negative stimulation effects occur. As an alternative, a binary search of stimulation amplitude can be performed. Should an effective amplitude not be obtained without negative stimulation effects, the electrode configuration can be changed (e.g., from monopolar to multi-polar) and the process of modifying amplitudes and testing stimulation can be repeated.

In various instances, modifications to electrode configuration can preceed modifications to stimulation patterns. For example, EES can be provided using different electrode configurations. If effective stimulation is not achieved from any of the electrode configurations, then the stimulation pattern may be changed (e.g., by changing the interburst interval, or the number of pulses in a burst, or the like). As may be appreciated, after each change of the stimulation pattern, multiple trials having different electrode configurations can be attempted. For each electrode configuration, multiple trials at different stimulation amplitudes can be performed.

In some instances, modifications to stimulation patterns can preceed modifications to electrode locations. As may be appreciated, after each change in electrode location, multiple stimulation patterns may be tested, as described above. Likewise, after each change of the stimulation pattern, multiple trials having different electrode configurations can be attempted. For each electrode configuration, multiple trials at different stimulation amplitudes can be performed.

The above exemplary ordering of modifications is not intended to be limiting. In some embodiments, modifications to stimulation patterns can be performed prior to changing electrode configurations. For example, EES can be applied using a single electrode configuration at varying combinations of stimulation amplitude and interburst interval (or with differing numbers of pulses per burst, interpulse interval, pulse shape, or the like). In some instances, the electrode configuration can then be changed and the process can be repeated.

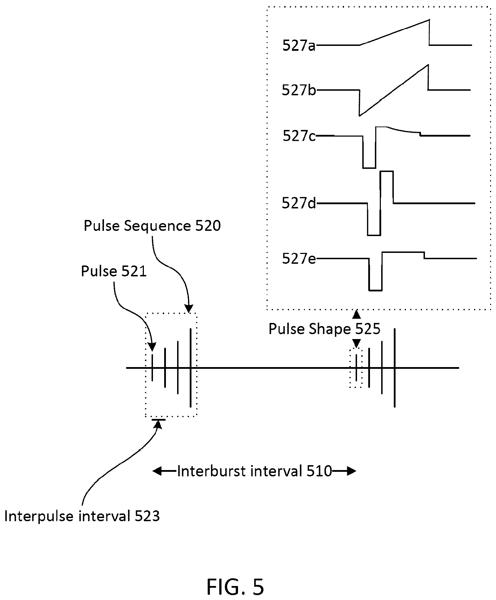

**FIG. 5** depicts exemplary stimulation waveforms, consistent with disclosed embodiments. The waveforms can be described in terms of an interburst interval 510 (or a corresponding interburst frequency), a pulse sequence 520, which includes one or more pulses (e.g., pulse 521). When the pulse sequence includes multiple pulses, then an interpulse interval 523 (or a corresponding interpulse frequency) can specify the delay between the pulses.

In some embodiments, the stimulation can be tonic stimulation. For such stimulation, the number of pulses in the pulse sequence can be 1. The interpulse interval 523 may be undefined, or equal to the interburst interval 510. In such embodiments, the interburst interval 510 can range from approximately 1 second to 8 ms, or less.

In some embodiments, the stimulation can be high-frequency stimulation. For such stimulation, the number of pulses in the pulse sequence can be 1. The interburst interval 510 may be undefined, or equal to the interpulse interval 523. In such embodiments, the interpulse interval 523 can range from approximately 50 ms to 100 ns, approximately 3 ms to 1.3 ms, or less.

In some embodiments, the stimulation can be burst stimulation. For such stimulation, the number of pulses in the pulse sequence can be greater than 1. The interburst interval 510 may be between 1 and 8 ms, or between 100 ms and 17 ms. The interpulse interval 523 can range from from approximately 50 ms to 100 ns, approximately 3 ms to 1.3 ms, or less. In some embodiments, the amplitudes of the pulses in the burst can vary. For example, pulse amplitudes can ramp up within a pulse (e.g., as depicted in **FIG. 5****).** The ramping can be linear or non-linear (e.g., exponential such that the amplitude of the pulse rises as *e^{t}* or 1 - *e^{-t}* or falls as *e^{-t},* or the like) Alternatively, the pulse amplitudes can be constant within a burst. In some embodiments, the pulse amplitudes can vary between bursts. For example, the amplitude can vary over the duration of the stimulation. For example, the maximum pulse amplitude in each burst can ramp up over a predetermined number of initial bursts (or ramp down over a predetermined number of final bursts).

A pulse can have a particular pulse shape 525. The disclosed embodiments are not limited to any particular pulse shape. As depicted in **FIG. 5****,** such pulses shapes can include monophasic or biphasic pulses. For example, pulse shape 527a depicts a monophasis ramp pulse. As an additional example, pulse shape 527b depicts a biphasic sawtooth pulse. Pulse shape 527b can be charge-balanced. As an additional example, pulse shape 527c depicts a biphasic pulse having a cathodic first pulse. The amplitude of the anodic repolarizing pulse is capped and decays exponentially for a predetermined time period. In some varients, the amplitude of the anodic repolarizing pulse, or the duration of the repolarizing pulse, is not limited. Pulse shape 527c can be charge-balanced. As an additional example, pulse shape 527d depicts a biphasic pulse having a cathodic first pulse and a symmetrical, charge-balanced anodic repolarizing phase. As a further example, pulse shape 527e depicts a biphasic pulse having a cathodic first pulse and an amplitude-limited, charge-balanced anodic repolarizing phase. The maximum amplitude of a pluse can be between 10 µa and 10 mA, depending on the application (e.g., nerve cuff electrodes may have microamp stimulation amplitudes, paddle electrodes may have micro-to-milliamp stimulation amplitudes, and carbon surface electrodes may have milliamp stimulation amplitudes).

The pulse can have particular duration(s). For example, in some embodiments the pulse can include a cathodic phase and an anodic phase, each with a duration (which may be the same duration). In some embodiments, the duration of the cathodic or anodic phases can be 10 µs to 1 ms. These durations can depend on the interpulse interval. In some embodiments, a duty cycle can be associated with the stimulation.

For example, when the stimulator is providing anodic or cathodic stimulation 50% of the time in a burst, then the duty cycle can be 50%. In some embodiments, the duty cycle can range from approximately 0.002% (e.g., 10 µs monophasic stimulation with an interpulse interval of 500 ms, or similar parameter combinations) to approximately 100% duty cycle (e.g., square wave, sine wave, triangle wave, or like stimulation).

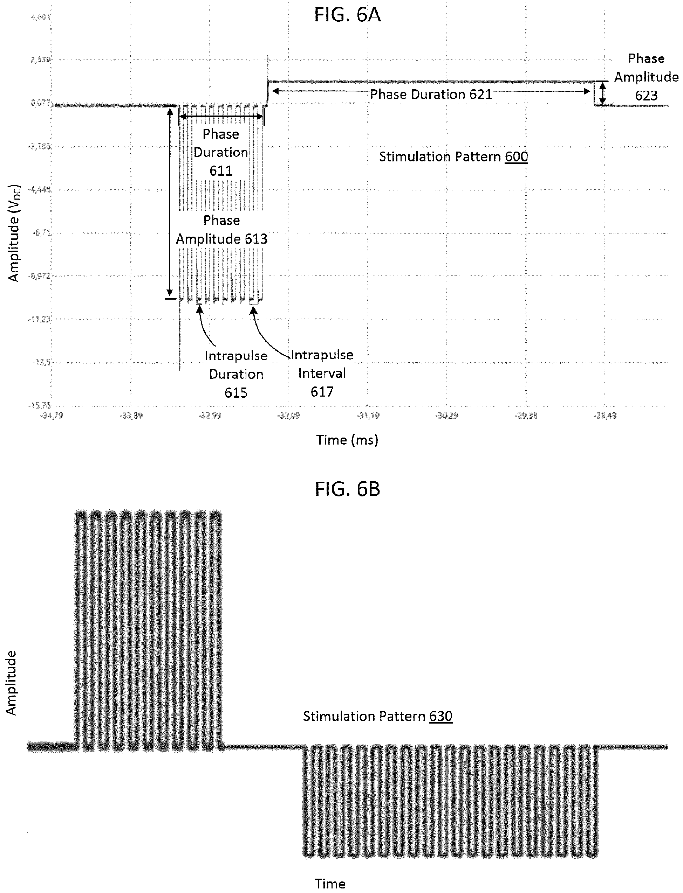

**FIGs. 6A** and **6B** depict stimulation patterns suitable for use with disclosed embodiments. These stimulation patterns superimpose a high-frequency modulating signal with one or both phases of a conventional biphasic stimulation pulse. In the two examples shown, the peak-to-peak amplitude of the high-frequency modulating signal for a phase is twice the amplitude of the conventional biphasic stimulation pulse for that phase, resulting in the phase comprising a set of monophasic pulses. However, the disclosed embodiments are not so limited. In some embodiments, the peak-to-peak amplitude of the high-frequency modulating signal can exceed, for a phase of the conventional biphasic stimulation pulse, twice the phase amplitude for the phase. The resulting phase can be a sequence of biphasic pulses with a non-zero DC offset. In various embodiments, the peak-to-peak amplitude of the high-frequency modulating signal can be less than twice the phase amplitude of the conventional biphasic stimulation pulse for that phase. The resulting phase can have a crenulated phase amplitude.

**FIG. 6A** depicts a trace of an exemplary stimulation pattern 600 for use in EES, consistent with some embodiments of the present disclosure. The trace depicted in **FIG. 6A** was obtained by providing current-controlled stimulation through two series resistors, a 100 ohm resistor and a variable resistor. Stimulation was provided using a multi-channel, current-controlled stimulator and the voltage across a 100 ohm resistor was measured using an oscilloscope.

In some embodiments, a stimulation pattern similar to stimulation pattern 600 can be included in a burst (e.g., similar to pulse 521). The burst can be separated from a prior or subsequent burst by an interburst interval (e.g., similar to interburst interval 510). The depicted stimulation pattern can be separated, in the burst, from a prior or subsequent pulse by an interpulse interval (e.g., similar to interpulse interval 523). The burst can include 1 or more pulses (e.g., with the maximum potential number of pulses in each burst depending the duration of each pulse, the interpulse interval, and the interburst interval).

A stimulation pattern similar to the depicted stimulation pattern 600 can include a first phase and a second phase. The first phase can have a phase duration 611 between 10 µs and 1 ms. The second phase can have a phase duration 621 between 10 µs and 10 ms. These durations can depend on the interpulse interval. The first phase can have a phase amplitude 613 between 10 µA and 10 mA. The second phase can also have a phase amplitude 623 between 1 µA and 10 mA. In some embodiments, the phase duration 621 may exceed phase duration 611, while phase amplitude 613 may phase amplitude 623. In some embodiments, phase amplitude 623 may be non-zero and less than 20%, or less than 10%, of phase amplitude 613. Phase amplitude 623 may be chosen to prevent the second phase of the stimulation pattern from having a physiological effect on the patient. The first and second phase may be charge-balanced. The first phase may be cathodic and the second phase anodic, or the second phase cathodic and the first phase anodic.

The first phase, the second phase, or both phases can include multiple instances of monophasic stimulation. For example, the first phase of stimulation pattern 600 includes 10 monophasic cathodic pulses. These stimulation pulses can have an intrapulse duration 615 (e.g., between 1 µs and 500 µs) and an intrapulse interval 617 (e.g., between 1 µs and 500 µs). Selecting values for two variables of the intrapulse duration 615, intrapulse interval 617, and number of pulses can determine the value of the remaining one of these variables.

**FIG. 6B** depicts an illustration of a further exemplary stimulation pattern 630 for use in EES, consistent with some embodiments of the present disclosure. Stimulation pattern 630 includes an anodic first phase and a cathodic second phase. Both the first phase and the second phase comprise multiple monophasic pulses. The first phase and the second phase are charge balanced, with the first phase having a greater phase amplitude than the second phase and the second phase having a greater phase duration than the first phase. As depicted in FIG. 6B, an interval can separate the first phase and the second phase of a stimulation pattern. The duration of this interval can be greater than zero and less than 1 ms.

The foregoing descriptions have been presented for purposes of illustration. They are not exhaustive and are not limited to precise forms or embodiments disclosed. Modifications and adaptations of the embodiments will be apparent from consideration of the specification and practice of the disclosed embodiments. For example, the described implementations include hardware, but systems and methods consistent with the present disclosure can be implemented with hardware and software. In addition, while certain components have been described as being coupled to one another, such components may be integrated with one another or distributed in any suitable fashion.

Moreover, while illustrative embodiments have been described herein, the scope includes any and all embodiments having equivalent elements, modifications, omissions, combinations (e.g., of aspects across various embodiments), adaptations or alterations based on the present disclosure. The elements in the claims are to be interpreted broadly based on the language employed in the claims and not limited to examples described in the present specification or during the prosecution of the application, which examples are to be construed as nonexclusive. Further, the steps of the disclosed methods can be modified in any manner, including reordering steps or inserting or deleting steps.

It should be noted that, the relational terms herein such as "first" and "second" are used only to differentiate an entity or operation from another entity or operation, and do not require or imply any actual relationship or sequence between these entities or operations. Moreover, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items.

The features and advantages of the disclosure are apparent from the detailed specification, and thus, it is intended that the appended claims cover all systems and methods falling within the true spirit and scope of the disclosure. As used herein, the indefinite articles "a" and "an" mean "one or more." Similarly, the use of a plural term does not necessarily denote a plurality unless it is unambiguous in the given context. Further, since numerous modifications and variations will readily occur from studying the present disclosure, it is not desired to limit the disclosure to the exact construction and operation illustrated and described, and accordingly, all suitable modifications and equivalents may be resorted to, falling within the scope of the disclosure.

As used herein, unless specifically stated otherwise, the term "or" encompasses all possible combinations, except where infeasible. For example, if it is stated that a stimulation pattern may include A or B, then, unless specifically stated otherwise or infeasible, the stimulation pattern may include A, or B, or A and B. As a second example, if it is stated that a stimulation pattern may include A, B, or C, then, unless specifically stated otherwise or infeasible, the stimulation pattern may include A, or B, or C, or A and B, or A and C, or B and C, or A and B and C.

It is appreciated that the above-described embodiments can be implemented by hardware or by a combination of hardware and software. If implemented in part by software, the software may be stored in the above-described computer-readable media. The software, when executed by a processor, can perform the disclosed methods. The computing units and other functional units described in this disclosure can be implemented by hardware, or software, or a combination of hardware and software. One of ordinary skill in the art will also understand that multiple ones of the above-described modules/units may be combined as one module/unit, and each of the above-described modules/units may be further divided into a plurality of sub-modules/sub-units.

In the foregoing specification, embodiments have been described with reference to numerous specific details that can vary from implementation to implementation. Certain adaptations and modifications of the described embodiments can be made. Other embodiments can be apparent to those skilled in the art from consideration of the specification and practice of the invention disclosed herein. It is intended that the specification and examples be considered as exemplary only, with a true scope and spirit of the invention being indicated by the following claims. It is also intended that the sequence of steps shown in figures are only for illustrative purposes and are not intended to be limited to any particular sequence of steps. As such, those skilled in the art can appreciate that these steps can be performed in a different order while implementing the same method.

In the drawings and specification, there have been disclosed exemplary embodiments. However, many variations and modifications can be made to these embodiments. Accordingly, although specific terms are employed, they are used in a generic and descriptive sense only and not for purposes of limitation or restriction of the scope of the embodiments, the scope being defined by the following claims

## Claims

1. A neuromodulation system, comprising:
a neuromodulation device,
a neuromodulation pattern storage means, and
a neuromodulation controller,
wherein the neuromodulation pattern storage means comprises neuromodulation data,
wherein the neuromodulation device provides neuromodulation according to the neuromodulation data, and
wherein the neuromodulation data specifies neuromodulation including a carrying frequency of at least 1 kHz.

2. The system of claim 1, wherein the neuromodulation device is an implantable pulse generator.

3. The system of claim 1, wherein the system further comprises at least one of a sensor, a programmer, a powering device, a lead comprising at least one electrode, a communication module, or a telemetry module.

4. The system of claim 1, wherein the system is configured to provide at least one of transcutaneous electrical stimulation, epidural electrical stimulation, subdural electrical stimulation, functional electrical stimulation, intramuscular stimulation, dorsal root ganglion stimulation, or peripheral nervous system stimulation.

5. The system of claim 1, wherein the carrying frequency is between 1 and 15 kHz.

6. The system of claim 1, wherein the neuromodulation data specifies at least one of a tonic, a burst, or a high-frequency waveform.

7. The system of claim 1, wherein the neuromodulation data specifies at least one of a ramp, mono-phasic, rectified, bi-phasic, exponential or sawtooth pulse shape.

8. The system of claim 1, wherein the neuromodulation data further specifies at least one of stimulation frequency, stimulation amplitude, stimulation current, pulse width, or electrode configuration.

9. The system of claim 1, wherein the neuromodulation data specifies multipolar stimulation.

10. A neuromodulation method, comprising:
equipping a patient with a neuromodulation device,
providing neuromodulation data to the neuromodulation device, and
providing neuromodulation to a patient using the neuromodulation device according to the neuromodulation data,
wherein the neuromodulation data specifies neuromodulation including a carrying frequency of at least 1 kHz.

11. The method of claim 10, wherein the neuromodulation device is an implantable pulse generator.

12. The method of claim 10, wherein the neuromodulation device comprises at least one of a sensor, a programmer, a powering device, a lead comprising at least one electrode, a communication module, or a telemetry module.

13. The method of claim 10, wherein the neuromodulation device is configured to provide at least one of transcutaneous electrical stimulation, epidural electrical stimulation, subdural electrical stimulation, functional electrical stimulation, intramuscular stimulation, dorsal root ganglion stimulation, or peripheral nervous system stimulation.

14. The method of claim 10, wherein the carrying frequency is between 1 and 15 kHz.

15. The method of claim 10, wherein the neuromodulation data specifies at least one of a tonic, a burst, or a high-frequency waveform.

16. The method of claim 10, wherein the neuromodulation data specifies at least one of a ramp, mono-phasic, rectified, bi-phasic, exponential or sawtooth pulse shape.

17. The method of claim 10, wherein the neuromodulation data specifies at least one of stimulation frequency, stimulation amplitude, stimulation current, pulse width, or electrode configuration.

18. The method of claim 10, wherein the neuromodulation data specifies multipolar stimulation.

19. The method of claim 10, wherein the neuromodulation is provided to treat pain of the patient.

20. The method of claim 10, wherein the neuromodulation data specifies a simulation pattern including a high-frequency modulating signal superimposed on one or both phases of a biphasic stimulation pulse.
